Europäisches Patentamt

European Patent Office

Office européen des brevets

(11) Veröffentlichungsnummer : **0 035 239**
**B1**

(12) # EUROPÄISCHE PATENTSCHRIFT

(45) Veröffentlichungstag der Patentschrift :
09.03.83

(51) Int. Cl.³ : **C 07 C128/00**

(21) Anmeldenummer : **81101385.3**

(22) Anmeldetag : **25.02.81**

(54) **Verfahren zur Herstellung von Guanidin-hydrochlorid.**

(30) Priorität : **28.02.80 DE 3007530**

(43) Veröffentlichungstag der Anmeldung :
**09.09.81 Patentblatt 81/36**

(45) Bekanntmachung des Hinweises auf die Patenterteilung : **09.03.83 Patentblatt 83/10**

(84) Benannte Vertragsstaaten :
**AT BE CH FR GB IT LI NL**

(56) Entgegenhaltungen :
**DE B 1 518 818**

**ULLMANNS « Encyklopädie der technischen Chemie », 4. Auflage, Band 12, 1976 VERLAG CHEMIE, Weinhelm, New York, Seiten 416, 417.**

**C. FERRI « Reaktionen der organischen Synthese », 1978 GEORG THIEME VERLAG, Stuttgart, Selten 662, 668.**

(73) Patentinhaber : **Degussa Aktiengesellschaft**
**Weissfrauenstrasse 9**
**D-6000 Frankfurt am Main 1 (DE)**

(72) Erfinder : **Schalke, Peter, Dr.**
**Südring 74**
**D-6458 Rodenbach (DE)**
Erfinder : **Langer, Manfred, Dr.**
**Stifterstrasse 30**
**D-8757 Karlstein (DE)**
Erfinder : **Huthmacher, Klaus, Dr.**
**Lärchenweg 18**
**D-6460 Gelnhausen (DE)**

Verfahren zur Herstellung von Guanidin-hydrochlorid

Die Erfindung betrifft ein Verfahren zur Herstellung von Guanidin-hydrochlorid.

Es ist bekannt, daß bei der Umsetzung von Dicyandiamid mit Ammoniumchlorid im Schmelzzustand Guanidin-hydrochlorid entsteht (DE-PS 752 750). Guanidin-hydrochlorid wird ebenfalls gebildet, wenn Dicyandiamid oder Cyanamid mit Ammoniumchlorid bei Temperaturen zwischen 80 und 200 °C in einem Lösungsmittel erhitzt wird, das im wesentlichen aus flüssigem Ammoniak besteht (US-PS 2 221 478). Auch durch Umsetzung von Cyanamid mit Ammoniumchlorid bei Temperaturen von 120 bis 170 °C in wässrigem Medium wird Guanidin-hydrochlorid erzeugt (DE-OS 1 593 472). Nachteilig ist bei diesen Verfahren, daß die benötigten Ausgangssubstanzen, das Dicyandiamid oder das Cyanamid, in einer aufwendigen mehrstufigen Umsetzung über das Calciumcyanamid aus Calciumcarbid gewonnen werden müssen.

Es ist außerdem bekannt, daß bei der Einwirkung von Chlorcyan auf Dimethylamin unter Druck bei Temperaturen von 130 bis 180 °C in einem mit Wasser nicht mischbaren organischen Lösungsmittel 1,1,2,2-Tetramethyl-guanidin entsteht (DE-PS 1 958 095) und bei der Einwirkung von Chlorcyan auf aromatische Amine in flüssiger Phase die entsprechenden Guanidin-Derivate sich bilden (DE-AS 1 518 818). Versuchtman, in entsprechender Weise durch Umsetzung von Chlorcyan mit Ammoniak Guanidin oder Guanidin-hydrochlorid herzustellen, so gelangt man nicht zu befriedigenden Ausbeuten.

Es wurde nun ein Verfahren zur Herstellung von Guanidin-hydrochlorid gefunden, das dadurch gekennzeichnet ist, daß Chlorcyan mit Ammoniak in Abwesenheit von Lösungsmitteln bei Temperaturen von 140 bis 220 °C umgesetzt wird und zu Beginn der Umsetzung ein Ammoniumsalz vorliegt. Dieses Verfahren ergibt hohe Ausbeuten an Guanidin-hydrochlorid. Das als Ausgangssubstanz benötigte Chlorcyan wird auf einfache Weise und mit fast quantitativer Ausbeute unmittelbar durch katalytische Umsetzung von Chlor mit Cyanwasserstoff gewonnen, ist also wesentlich leichter zugänglich als die für die bekannten Verfahren zur Herstellung von Guanidin-hydrochlorid erforderlichen Ausgangssubstanzen.

Bei der Durchführung des erfindungsgemäßen Verfahrens ist es im allgemeinen zweckmäßig, das stöchiometrische Verhältnis Ammoniak zu Chlorcyan — 2 Mol Ammoniak je Mol Chlorcyan — nicht wesentlich zu unterschreiten, nämlich nicht weniger als etwa 1,5 Mol Ammoniak je Mol Chlorcyan anzuwenden. Wenngleich andererseits das Verhältnis beliebig groß gewählt, das heißt Ammoniak in beliebigem Überschuß genommen werden kann, ist es im allgemeinen zweckmäßig, nicht mehr als etwa 10 Mol Ammoniak je Mol Chlorcyan einzusetzen. Vorzugsweise beträgt das molare Verhältnis Ammoniak zu Chlorcyan 2,0 zu 5,0, insbesondere 2,1 zu 3,5.

Erfindungsgemäß erfolgt die Umsetzung in Abwesenheit von Lösungsmitteln, insbesondere von organischen Lösungsmitteln, jedoch kann es in manchen Fällen, beispielsweise zur leichteren Abführung der bei der Umsetzung auftretenden Wärme, vorteilhaft sein, dem Umsetzungsmedium Inertgase, wie Stickstoff oder Luft, zuzuführen. Im Sinne der Inertgase kann auch Ammoniak dienen, und es wird von diesem gegebenenfalls ein großer Überschuß, zweckmäßigerweise jedoch nicht mehr als etwa das 50 fache der stöchiometrisch erforderlichen Menge, eingesetzt.

Es ist zweckmäßig, zu Beginn der Umsetzung ein Ammoniumsalz, insbesondere Ammoniumchlorid, zuzusetzen. Wenngleich es sich um weitgehend beliebige Mengen Ammoniumsalz handeln kann, ist es im allgemeinen vorteilhaft, daß 0,01 bis 0,10 Mol, insbesondere 0,02 bis 0,05 Mol, Ammoniumsalz je Mol Chlorcyan vorliegen. Anstelle des Ammoniumsalzes kann selbstverständlich, falls eine entsprechende Menge überschüssigen Ammoniaks verfügbar ist, eine Säure, insbesondere Chlorwasserstoff, eingesetzt werden.

Für die Umsetzung kommen im allgemeinen Temperaturen von 140 bis 220 °C, vorzugsweise von 160 bis 200 °C, insbesondere von 175 bis 200 °C, in Frage. In manchen Fällen kann es vorteilhaft sein, bei niedrigeren Temperaturen, beispielsweise bei Temperaturen zwischen − 40 und 0 °C, zu beginnen, daß heißt, die Umsetzungsteilnehmer bei diesen Temperaturen miteinander in Berührung zu bringen und dann auf die eigentliche Umsetzungstemperatur von 140 bis 220 °C zu erhitzen.

Die Umsetzung kann an sich bei beliebigen Drücken durchgeführt werden, jedoch ist es, damit einfache Apparate verwendet werden können, im allgemeinen zweckmäßig, bei Normaldruck oder allenfalls geringfügig erniedrigtem oder erhöhtem Druck, bis etwa 5 bar, zu arbeiten.

Für das erfindungsgemäße Verfahren kommt sowohl eine diskontinuierliche als auch eine kontinuierliche Arbeitsweise in Frage. Bei der kontinuierlichen Arbeitsweise ist es vorteilhaft, einen Reaktor in Form eines Strömungsrohrs zu verwenden und an dessen Eingang die Ausgangssubstanzen so zuzuführen, daß sie sofort innig miteinander vermischt werden, ferner Anteile des Umsetzungsgemischs, beispielsweise unter Verwendung eines Schlaufenreaktors, im Kreislauf zu führen.

Das bei der Umsetzung anfallende Guanidin-hydrochlorid kann von den gegebenenfalls enthaltenen Verunreinigungen in bekannter Weise befreit werden, zum Beispiel von Ammoniumchlorid durch Zugabe einer entsprechenden Menge Guanidin-carbonat.

Beispiel 1

Es wurden in einem Kolben 2,9 g (0,05 Mol) Ammoniumchlorid vorgelegt. In den Kolben wurden dann im Verlauf von einer halben Stunde 67,0 g (1,09 Mol) Chlorcyan und 56,1 g (3,30 Mol) Ammoniak gleichzeitig gasförmig eingeleitet. Hierbei wurde der Kolbeninhalt ständig auf einer Temperatur von 180 bis 205 °C gehalten und das überschüssige Ammoniak laufend abgetrieben. Schließlich wurde das Umsetzungsgemisch weitere zwei Stunden lang unter Rühren auf 180 °C gehalten. Das Produkt — 98 g — bestand zu 91 % aus Guanidin-hydrochlorid. Die Ausbeute an Guanidin-hydrochlorid, bezogen auf eingesetztes Chlorcyan, betrug 86 %.

Beispiel 2

In ein Strömungsrohr wurden stündlich in gleichförmigem Strom 22 g (0,6 Mol) Chlorwasserstoff, 753 g (12 Mol) Chlorcyan und 238 g (14 Mol) Ammoniak eingedüst. Im Inneren des Strömungsrohrs wurde die Temperatur auf 190 °C gehalten. Das Umsetzungsgemisch trat nach einer mittleren Verweilzeit von 30 Minuten aus dem Strömungsrohr aus. Das nicht umgesetzte Ammoniak wurde abgetrennt und dann wurde das Umsetzungsgemisch in einem zweiten Strömungsrohr bei einer mittleren Verweilzeit von 3 Stunden weiter auf 190 °C gehalten. Nach Austritt aus dem Strömungsrohr und Abkühlung erstarrte das Umsetzungsgemisch kristallin. Es enthielt 96 % Guanidin-hydrochlorid und 3 % Ammoniumchlorid. Stündlich wurden 1 196 g gewonnen, entsprechend einer Ausbeute an Guanidin-hydrochlorid von 98 %, bezogen auf eingesetztes Chlorcyan.

## Ansprüche

1. Verfahren zur Herstellung von Guanidin-hydrochlorid, dadurch gekennzeichnet, daß Chlorcyan mit Ammoniak in Abwesenheit von Lösungsmitteln bei Temperaturen von 140 bis 220 °C umgesetzt wird und zu Beginn der Umsetzung ein Ammoniumsalz vorliegt.

2. Verfahren nach Anspruch 1, dadurch gekennzeichnet, daß 2,0 bis 5,0 Mol Ammoniak je Mol Chlorcyan angewendet werden.

## Claims

1. A process for the production of guanidine hydrochloride, characterised in that cyanogen chloride is reacted with ammonia in the absence of solvents at a temperature of from 140 to 220 °C, and an ammonium metal salt is present at the beginning of the reaction.

2. A process according to claim 1, characterised in that from 2.0 to 5.0 mols of ammonia are used per mol of cyanogen chloride.

## Revendications

1. Procédé pour la fabrication de chlorhydrate de guanidine, caractérisé en ce que l'on fait réagir du chlorure de cyanogène avec du gaz ammoniac, en l'absence de solvant, à une température comprise entre 140 et 220 °C, et que pour démarrer la réaction, on prévoit la présence d'un sel d'ammonium.

2. Procédé selon la revendication 1, caractérisé en ce que l'on utilise 2 à 5 moles d'ammoniac par mole de chlorure de cyanogène.